(19) Europäisches Patentamt
European Patent Office
Office européen des brevets

(11) Publication number: **0 074 264**
**B1**

# (12) EUROPEAN PATENT SPECIFICATION

(45) Date of publication of patent specification: **11.05.88**

(51) Int. Cl.$^4$: **A 61 K 7/08**

(21) Application number: **82304664.4**

(22) Date of filing: **06.09.82**

## (54) Shampoo.

(30) Priority: **08.09.81 US 300078**

(43) Date of publication of application:
**16.03.83 Bulletin 83/11**

(45) Publication of the grant of the patent:
**11.05.88 Bulletin 88/19**

(84) Designated Contracting States:
**AT BE CH DE FR GB IT LI NL SE**

(56) References cited:
**EP-A-0 018 717**
**FR-A-1 576 613**
**US-A-3 149 042**
**US-A-3 964 500**

(73) Proprietor: **UNILEVER PLC**
**Unilever House Blackfriars P.O. Box 68**
**London EC4P 4BQ (GB)**
(84) **GB**

(73) Proprietor: **UNILEVER NV**
**Burgemeester s'Jacobplein 1 P.O. Box 760**
**NL-3000 DK Rotterdam (NL)**
(84) **BE CH DE FR IT LI NL SE AT**

(72) Inventor: **Pader, Morton**
**1358 Sussex Road**
**Teaneck New Jersey (US)**

(74) Representative: **Doucy, Robert Henry et al**
**Unilever PLC Patents Division P.O. Box 68**
**Unilever House**
**London EC4P 4BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to aqueous liquid hair conditioning shampoos. More particularly this invention relates to aqueous liquid hair conditioning shampoos comprising an anionic and/or amphoteric detergent, water and a non-ionic and/or cationic hair conditioning agent.

Applicant has found that the inclusion in such shampoos of from 10 to 70% by weight of a water-miscible saccharide advantageously improves the foam quality of the shampoo, more especially the foam volume and/or foam viscosity. The saccharide also enhances the stability of the hair conditioning agent in the shampoo.

The use of saccharides in liquid hair shampoos has previously been mentioned in US Patents Nos. 3 998 761, 3 988 438, 3 149 042 and 2 237 629 and their inclusion in other hair cosmetic compositions is referred to in European Patent application No. 5807. Also, FR—A—1 576 613 concerns the addition of small amounts of a sugar not exceeding 2 g/l (i.e. 0.2% by weight) to surfactants, in order to enhance their foaming power. It has however not previously been reported that saccharides in the relatively large amounts disclosed herein can be employed to enhance the foam quality of conditioning shampoos, and also to stabilise the hair conditioning agent in the shampoo.

According to the present invention there is provided an aqueous liquid hair conditioning shampoo composition comprising:

(a) from 3 to 60% by weight of an anionic or amphoteric detergent or a mixture thereof;

(b) from 0.1 to 30% by weight of at least one cationic or non-ionic hair conditioning agent;

(c) from 20 to 75% by weight water; and

(d) from 10 to 70% by weight of a saccharide chosen from mono-, di- and polysaccharides, their hydrogenation products and mixtures thereof, which are completely miscible with water in that a mixture of 70 parts by weight saccharide and 30 parts by weight water will yield an essentially clear solution which is pourable at 0°C or higher.

The shampoo of the invention employs a known anionic or amphoteric detergent or mixture thereof. Suitable anionic detergents are the alkyl sulphates, alkyl ether sulphates, alkaryl sulphonates, alkyl succinates, alkyl sulphosuccinates, N-alkanoyl sarcosinates, and alpha-olefin sulphonates. Especially preferred are the sodium, magnesium, ammonium, and the mono-, di- and triethanolamine salts of alkyl and alkaryl sulphates as well as these salts of alkaryl sulphonates. The alkyl groups of the detergents generally have a total of from 12 to 21 carbon atoms, may be unsaturated, and are preferably fatty alkyl groups. The sulphates may be sulphate ethers containing one to ten ethylene oxide or propylene oxide units per molecule. Preferably, the sulphate ethers contain 2 to 3 ethylene oxide units.

Typical anionic detergents include sodium lauryl sulphate, sodium oleyl succinate, ammonium lauryl sulphosuccinate, sodium lauryl ether sulphate, ammonium lauryl sulphate, sodium dodecylbenzene sulphonate, triethanolamine dodecylbenzene sulphonate and sodium N-lauroyl sarcosinate. The most preferred anionic detergents are the lauryl sulphates, particularly monoethanolamine, triethanolamine and ammonium lauryl sulphates. Sodium lauryl ether sulphate is also very suitable for use in the compositions of the invention.

Suitable amphoteric detergents for use in the shampoos of the invention include N - lauryl - N' - carboxymethyl - N' - (2 - hydroxyethyl)ethylenediamine, coco-betaine, and the Miranol® compounds in US Patent Nos. 2 528 378 and 2 781 354. Other suitable amphoteric detergents include the quaternary cycloimidates, betaines, and sultaines disclosed in US Patent No. 3 964 500. Particularly preferred amphoteric surfactants are the substituted quaternary hydroxy cycloimidinic acid alkali metal alcoholates described in US Patent No. 2 528 378. The most preferred of the amphoteric surfactants are the substituted quaternary hydroxy cycloimidinic acid alkali metal alkoxymethyl carboxylates described in US Patent No. 2 781 354. Specific betaines useful in the shampoos of the invention are for example alpha - (tetradecyldimethylammonio) acetate, beta - (hexadecyldiethylammonio) propionate, and gamma - (dodecyldimethylammonio) butyrate. Examples of suitable sultaines are—3 - (dodecyldimethylammonio) propane - 1 - sulphonate, and 3 - (tetradecyldimethylammonio) ethane - 1 - sulphonate.

The detergent system of the shampoo of the invention may additionally include a nonionic detergent such as a fatty acid alkanolamide or an alkylene oxide (ethylene oxide and propylene oxide) condensate of a hydrophobic base such as a long chain fatty alcohol or an alkylphenol. Typical of the fatty acid alkanolamides are those having a total of from 10 to 21 carbon atoms, such as lauric diethanolamide, coconut oil monoethanolamide and lauric isopropanolamide. The alkylene oxide condensates of long chain fatty alcohols include $C_{10}$ to $C_{21}$ fatty alcohols condensed with 3 to 20 moles of ethylene oxide, such as the ethylene oxide condensates of lauryl alcohol, myristyl alcohol and palmityl alcohol. The alkylene oxide condensates of alkylphenols include the alkylphenols having a $C_8$ to $C_{15}$ alkyl group condensed with 3 to 20 moles of ethylene oxide, such as the octylphenol-8 mole ethylene oxide condensate, and the nonylphenol-10 mole ethylene oxide condensate.

Preferably the surfactant system is present in an amount from 7 to 40% and most preferably from 10 to 30% by weight of the shampoo composition.

The shampoo of the invention also comprises a cationic or non-ionic conditioning agent which are known ingredients of shampoos. Cationic hair conditioning agents include the cationic cellulose ethers described in US Patents Nos. 3 816 616 and 4 272 515 and which are available commercially from Union

Carbide Corp. under the trade mark Polymer JR. Other suitable materials are the cationic polygalactomannan gum derivatives described in US Patent No. 4 298 494 which are commercially available under the trade mark Jaguar from Celanese-Stein Hall. An example of a suitable material is the hydroxypropyltrimethylammonium derivative of guar gum of the formula

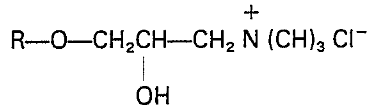

where R represents guar gum. Such a material is available under the name Jaguar®C-13-S. This material also has the CTFA designation Guar Hydroxypropyltrimonium Chloride. In Jaguar®C-13-S the degree of substitution of the cationic groups is about 0.13. Another suitable material is that known as Jaguar®C-17 which is similar to Jaguar®C-13-S but has a higher degree of substitution of cationic groups of about 0.25—0.31. A further example of a suitable guar derivative is the hydroxypropylated cationic guar derivative known as Jaguar®C-16 which as well as containing the above cationic quaternary ammonium groups also contains hydroxypropyl ($—CH_2CH(OH)CH_3$) substituent groups. In Jaguar®C-16 the degree of substitution of the cationic groups is 0.11—0.16 and the moles of substitution of hydroxypropyl groups is 0.8—1.1.

Other cationic conditioning agents useful in the shampoos of the present invention include cationic polyamide polymers such as the low molecular weight adipic acid/diethylene-triamine polyamide and the copolymers of vinylpyrrolidone and dimethylaminoethyl methacrylate quaternised with dimethyl sulphate (Gafquat 755, GAF Corporation) described in US Patent No. 4 080 310; the graft cationic copolymer containing N-vinylpyrrolidone, dimethylaminoethyl methacrylate and polyethylene glycol described in US Patent 4 048 301; the mineral acid salts of the amino-alkyl esters of homo- and copolymers of unsaturated carboxylic acids having from 3 to 5 carbon atoms described in US Patent No. 4 009 256; and the polymers of etherified starch described in US Patent No. 3 186 911.

The high molecular weight polymers sold under the trade mark Merquat® by Merck & Co., Inc., are cationic polymers which are also suitable for use in the present shampoos. Representative ones are Merquat 100, a highly charged cationic dimethyldiallylammonium chloride homopolymer, and Merquat®550, a highly charged cationic copolymer prepared with dimethyldiallylammonium chloride and acrylamide. These materials are designated in the CTFA dictionary as Quaternium-40 and Quaternium-41, respectively.

Insoluble hair conditioning agents can be present in the shampoo composition as colloidal dispersions or emulsions, or as suspensions. These hair conditioning agents may be cationic or nonionic. Most insoluble hair conditioning agents are nonionic.

Suitable nonionic hair conditioning agents are described in US Patent No. 3 932 610 and 3 533 955 and include silicones, resinous materials, waxy materials, and oily materials.

Silicones are the preferred insoluble hair grooming agents. The use of silicones in shampoos has been described in US Patent Nos. 2 826 551 and 3 964 500.

Suitable silicones include polyalkyl or polyaryl siloxanes with the following structure:

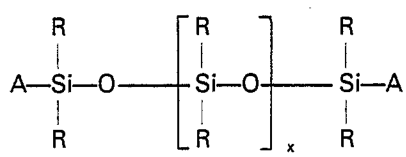

wherein R is alkyl or aryl, and x is an integer from 100 to 2,400. A represents groups which block the ends of the silicone chains. Suitable A groups include methyl, methoxy, ethoxy, propoxy, and aryloxy. The two R groups on the silicone atom may represent the same group or different groups. Preferably the two R groups represent the same group. Suitable R groups include methyl, ethyl, propyl, phenyl, methylphenyl and phenylmethyl. The preferred silicones are polydimethyl siloxane, polydiethylsiloxane, and polydimethylphenylsiloxane. Polydimethylsiloxane is especially preferred.

Other silicones suitable for use in the present invention include the cyclic silicones. These materials have the formula

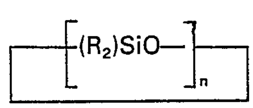

where n is 4 or 5 and R has the same meaning as in the structure of linear siloxanes.

The dimethyl cyclic silicones are volatile, and are thus present on the hair only temporarily. They are, therefore, especially preferred since they provide lubricity and other desirable qualities to the hair, as do their linear-chain counterparts, and then evaporate. Volatile cyclic silicones are available under the trade name Dow Corning 344 and 345 fluids from the Dow Corning Corporation.

The silicones useful in the present shampoos may also be modified by adding polyethers as copolymers as is described in US Patent No. 3 957 970. Such copolymers tend to be more soluble than homopolymers of silicone.

Various other nonionic hair conditioning agents may be used, including wood rosins and their $C_1$—$C_6$ esters, as described in US Patents Nos. 3 932 610 and 3 950 510.

Oily nonionic hair grooming agents may also be used, such as those mentioned in US Patent No. 3 533 955.

A particularly preferred hair conditioning agent system is a mixture of a silicone and a cationic cellulose, for instance a mixture of a polydimethylsiloxane and a Polymer JR resin.

The hair conditioning agent, or mixture of hair conditioning agents, should be present in an amount from 0.1 to 30% by weight, preferably 0.2 to 10% and most preferably 0.3 to 5% by weight of the shampoo composition.

The water concentration of the shampoo is between 20 and 75% by weight. Preferably the amount of water is from 30 to 60% by weight and most preferably from 40 to 60%.

It has surprisingly been found that the inclusion in the shampoo of certain saccharides stabilises the shampoo and at the same time enhances the foam quality. Thus, the presence of a saccharide in a shampoo can increase the foam quality at a given concentration of detergents, or can maintain the same level of foam quality at a lower detergent concentration. At the same time, the saccharide thickens the shampoo and stabilises the shampoo, either by helping to dissolve the hair conditioning agent, or by keeping in suspension those hair conditioning agents which are insoluble. The mechanism for the enhancement of foam quality is not known.

In the present specification, the term saccharide includes mono-, di- and polysaccharide molecules as well as hydrogenation products and mixtures thereof. Suitable monosaccharides include glucose, dextrose, fructose and xylose. Preferably the monosaccharide is fructose or glucose. Suitable disaccharides include sucrose, maltose and lactose.

Suitable polysaccharides include chains containing three or more units of glucose. While polysaccharides generally contain chains comprising mainly glucose, it is also possible for saccharides other than glucose to be present in the chain.

The upper limit of the number of units of a polysaccharide or the average number of units of a mixture of polysaccharides depends on the desired viscosity of the shampoo and on the concentration of saccharides in the shampoo at that viscosity. To achieve a given viscosity, the higher the concentration of saccharide the smaller the number of units per saccharide molecule. For example, a smaller amount of a twenty dextrose equivalent corn syrup than of a sixty dextrose equivalent corn syrup is required to achieve a given viscosity in a given shampoo system.

A further limitation of the number of units per saccharide molecule is the requirement that the saccharide be completely miscible with water. By miscible it is meant that a mixture of about 70 parts by weight saccharide and 30 parts by weight water will yield an essentially clear solution which is pourable at 0°C or higher. Water miscibility decreases with increasing number of units per saccharide molecule.

It will frequently be desirable to employ hydrogenated saccharides equivalent in structure to the non-hydrogenated saccharides from which they were derived. This allows for greater shampoo stability with respect to colour and aroma. The absence of a reducing group in hydrogenated saccharides enhances stability of the molecule to fermentation by micro-organisms and to such reactions as the browning reaction. Suitable hydrogenated saccharides include those derived from the hydrolysis of starches such as sorbitol and mixtures of sorbitol, maltitol, and the hydrogenated form of polysaccharides such as maltitriol and maltitetraol. Suitable methods for hydrogenating saccharides include those described in US Patent No. 3 329 507, DOS 2 008 865 and DOS 2 008 865.

It is normally very difficult to separate saccharides from one another. In commercial practice, therefore, a mixture of saccharides is used advantageously. Suitable mixtures of saccharides include syrups obtained by hydrolysing carbohydrates. The hydrolysis may be catalysed chemically or enzymatically or both as described in US Patent No. 3 329 507.

The carbohydrates hydrolysed may be starch or cellulose. Starch hydrolyzates are particularly desirable because they are readily available, relatively inexpensive, relatively easy to prepare, and obtainable from a wide variety of sources such as corn, potato, wheat and rice.

A preferred saccharide for use in the present invention is corn or potato syrup.

The hydrolysis of the carbohydrates may be partial or complete. Complete hydrolysis leads to glucose. Partial hydrolysis leads to a mixture of saccharides. Such a mixture may contain monosaccharides, disaccharides and/or polysaccharides.

The saccharides used in shampoo compositions of the present invention desirably have dextrose equivalents of 20 to 100, preferably 30 to 100 and most preferably 30 to 70. Some suitable syrups for use in the present invention include corn syrups with about 20, 43 and 60 dextrose equivalent. Also useful is the so-called high fructose syrup made by the enzymatic conversion of glucose to fructose as well as the residues from various sugar and corn syrup refining processes. The saccharides of the present invention are clearly distinguishable from unhydrolysed cellulosic gums used to thicken some prior art shampoos. As is also the case with starch, cellulosic gums fall outside the miscibility range cited above. In addition, cellulosic gums provide high thickening at low concentration. For example, a 2% solution of a cellulosic

gum such as sodium carboxymethylcellulose in water will yield an essentially non-pourable solution. A 2% solution of a saccharide used in this invention, on the other hand, will change the viscosity of water only minimally. The resulting solution will flow readily.

The shampoos of the present invention contain 10 to 70%, preferably 15 to 60% and most preferably 20 to 50%, by weight of a suitable saccharide. Concentrations of saccharides below the lower limit are outside the present invention since they do not provide sufficient foam quality enhancement or thickening. Concentrations of saccharides above the upper limit are undesirable because they may lead to shampoos which are too viscous and because they are economically disadvantageous. It is, however, not suggested that high levels will not provide the enhancement of foam quality realised with the lower levels. Levels higher than the upper limit simply serve no purpose and only add to the expense of formulating the shampoo.

Adjuvants may optionally be added to the shampoo composition, for example an antibacterial agent to help preserve the system such as formaldehyde, imidazolidinyl urea, glutaraldehyde and other available products, e.g. Dowicil®200 offered by the Dow Chemical Group.

Other adjuvants may be added to inhibit darkening or other colour deterioration of the shampoo. Ordinarily, such are not necessary. When the shampoo comprises certain reducing substances, as when it comprises corn syrup, it may be valuable to add an inhibitor of the so-called "browning reaction" which is well known in food technology. This reaction involves the free aldehydic groups of carbohydrates. It is markedly reduced by the addition of sulphite or bisulphite ions and by maintaining a relatively low pH. Thus, shampoos of this invention comprising corn syrup or other reducing sugars should preferably not be excessively alkaline if they are to have a good shelf life, the preferred pH being below 8. Also, they will advantageously contain enough of a sulphite source as to provide 100 ppm or more sulphite or bisulphite as $SO_2$.

Further adjuvants include colouring agents and perfuming agents. A monohydric, dihydric or trihydric alcohol, such as ethyl alcohol, propylene glycol or glycerol, may be included in the shampoo composition to assist dispersion or dissolution of the hair conditioning agent.

Soaps may be added to the surfactant systems in the shampoos. The soaps usable herein are well-known alkali metal, ammonium or substituted ammonium salts such as triethanolamine salts of natural or synthetic fatty acids having 12 to 20 carbon atoms. The fatty acids may be saturated or unsaturated and may be derived from vegetable oils such as coconut oil, peanut oil, rapeseed oil and corn oil, or from animal sources such as tallow and lard. The alkali metals are preferably sodium or potassium. Ammonium ion may also be used. Suitable soaps include sodium or triethanolamine laurate-myristate or sodium or triethanolamine oleate.

In view of the unexpectedly superior foam enhancing and shampoo stabilising properties of the saccharides used in the present invention, it is frequently not necessary to include additional foam enhancers and shampoo thickeners, but they may be added if desired.

The shampoo compositions of the invention will generally have a viscosity of from $4\times10^{-4}$ to $6\times10^{-3}m^2/s$ (400 to 6,000 centistokes) at 25°C, as measured with a Brookfield viscometer, so that they are satisfactorily pourable. Additional thickeners may be included if the desired shampoo viscosity is not obtained by the presence of the saccharide. In such cases, conventional thickeners may be used to increase the viscosity of the shampoo to the desired level. Suitable thickeners include carboxymethyl cellulose, methyl cellulose, hydroxypropyl cellulose, vinyl carboxy polymers available under the name Carbopol® (Goodrich Co.), vegetable gums, alginates and derivatives thereof, and latexes. A particularly suitable viscosity range is $5\times10^{-4}$ to $4.5\times10^{-3}m^2/s$ (500 to 4500 centistokes), more particularly to $10^{-3}$ to $4\times10^{-3}m^2/s$ (1000 to 4000 centistokes) at 25°C.

Various fatty acid amides may be used to obtain specific foam characteristics and to thicken the shampoo. Suitable amides include coconut fatty acid diethanolamide and lauric isopropanolamide. These amides are usually not necessary, since the saccharide can be made to fulfil their function. They may also be undesirable because they add to the cost of the shampoo, depending on the relative costs of the amide and the saccharide and their levels of incorporation.

There will now be described the experimental procedure employed to obtain the foam volume and foam viscosity values for specific shampoos given below.

The shampoo to be tested was diluted with water at approximately the dilution used in shampooing in vivo. The diluted shampoo was then whipped in a household food mixer under specific conditions. The volume of foam was then measured by determining the density of the foam generated and calculating the volume of the foam therefrom. The viscosity of the foam was measured by determining the time (in seconds) required to force the foam a specified distance through a capillary tube by air pressure produced by a plunger. The time required is a measure of the foam viscosity.

The apparatus for generating the foam consisted of a household food mixer equipped with a mixing bowl and two metal whisk-type beaters rotating in intersecting circles. The apparatus for measurement of foam viscosity consisted of an aluminium cup into which the foam was placed, a metal cap to fit over the cup, a capillary tube protruding from the cup, and a plunger which forced air into the cup to push the foam up the capillary tube. The cylindrical cup was 27 mm wide×55 mm high. The tube was 30 cm long with 1 cm internal diameter. It had a calibration mark 25 mm from the top. An opening in the centre of the metal cap accommodated the capillary tube, the bottom of which extended 20 mm below the bottom edge of the

metal cap. A rubber grommet formed an airtight seal between the cap orifice and the wall. The metal cap also contained an air inlet to accept forced air from a plunger, which consisted of a metal cylindrical piston which moved freely through a glass barrel. The barrel was mounted vertically within an electric vibrating unit, which prevented the piston from stopping during its descent. To an outlet at the bottom of the barrel was affixed one end of plastic tubing. The other end was affixed to the air inlet on the metal cap.

Measurement of specific foam volume was accomplished through the use of a plastic Petri dish and a double pan torsion or a single pan electronic balance with readout capacity to the nearest 0.01 g.

The test solution was prepared as follows: Thirty ml of the test shampoo were diluted with 330 ml of tap water and mixed gently with a magnetic stirrer to avoid foaming. Fifty ml of this solution was transferred to the mixing bowl of the food mixer. The solution was then mixed at a predetermined speed (700 rpm) for exactly three minutes, by which time all the solution was converted to foam. The mixer was stopped, and immediately 20 ml of foam were collected using a 20 ml glass syringe from which the shoulder and neck had been removed. The collected foam was transferred to the bottom half of the pre-weighed Petri dish. The top half was put into place over the bottom half and the dish was set aside for weighing. Another 20 cc of foam were quickly collected and transferred to the cylindrical cup. The piston of the plunger unit was raised to its maximum height and the vibrating device was activated. The metal cap with capillary tube was fitted tightly onto the cup. A finger was placed over the top of the capillary tube to prevent the piston from descending prematurely. An electric timer with readability to the nearest 0.1 s was used to measure the viscosity. The timer was activated at the exact moment at which the finger was removed from the top of the capillary tube. The piston moved downward, forcing air into the foam cup and pushing the foam up the tube. When the foam reached the calibration mark on the capillary tube, the time was noted. The elapsed time was taken as the foam viscosity.

The Petri dish containing the 20 ml of foam was weighed. The difference between the weight of the Petri dish alone and that of the Petri dish plus foam was the weight in grams of the foam. To determine the specific foam volume in ml/g, the volume of foam, 20 ml, was divided by the foam weight. Thus, foam volume, as given below, represents the volume of foam per unit weight of diluted shampoo.

A minimum of three replicates was used for each shampoo tested. Data were compared only within tests run as a series; series run at different times were not intercompared because of variations in such uncontrollable factors as mixer speed and operator variability. The precision in the foam volume measurements was ±4% and that in the foam viscosity measurement was ±7%.

The Examples given below illustrate the invention. Percentages are by weight.

Example I

The following series of formulations were prepared and evaluated by the in vitro foam test.

| | % | | | | |
|---|---|---|---|---|---|
| Shampoo: | A | B | C | D | E |
| Triethanolamine lauryl sulphate | 10.0 | 10.0 | 10.0 | 10.0 | 10.0 |
| Hydroxypropylmethylcellulose[1] | 0.2 | 0.2 | 0.2 | 0.2 | 0.2 |
| Cocodiethanolamide | — | — | 1.0 | — | 1.0 |
| Corn syrup (80% solids)[2] | — | — | — | 40.0 | 40.0 |
| Polydimethylsiloxane[3] | — | 2.0 | 2.0 | 2.0 | 2.0 |
| Water | to 100 | to 100 | to 100 | to 100 | to 100 |
| Foam volume (ml/g) | 9.9 | 8.9 | 9.2 | 10.8 | 9.9 |
| Foam viscosity (sec) | 4.7 | 4.4 | 4.3 | 5.7 | 4.7 |

[1] Methocel E4M® (Dow Chemical).
[2] 42 Dextrose equivalent (Staley®1300).
[3] Viscosity of $6\times10^{-2} m^2/s$ (60,000 centistokes) (Viscasil®, General Electric Co.).

It will be seen that whereas the inclusion of the silicone had a negative effect on foam quality (see Shampoo B) the inclusion of the saccharide improved foam quality (see Shampoo D). The properties of Shampoo D were not improved by the further inclusion of cocodiethanolamide (see Shampoo E).

Example II

The following series of formulations were made and tested:—

| | % | | | | |
|---|---|---|---|---|---|
| Shampoo: | A | B | C | D | E |
| Alpha-olefin sulphonate[1] | 10.0 | 10.0 | 10.0 | 10.0 | 10.0 |
| Hydroxypropylmethylcellulose[2] | 0.2 | 0.2 | 0.2 | 0.2 | 0.2 |
| Cocodiethanolamide | — | — | 1.0 | — | 1.0 |
| Corn syrup (80% solids)[3] | — | — | — | 40.0 | 40.0 |
| Polydimethylsiloxane[4] | — | 2.0 | 2.0 | 2.0 | 2.0 |
| Water | to 100 | to 100 | to 100 | to 100 | to 100 |
| Foam volume (cc/g) | 9.9 | 9.5 | 9.0 | 10.2 | 10.6 |
| Foam viscosity (sec) | 4.1 | 4.6 | 4.2 | 5.3 | 5.7 |

(1) Sulphramin®AOS, Witco Chemical Corp..
(2) As in Example I.
(3) As in Example I.
(4) As in Example I.

The results for Shampoos D and E show the beneficial effect of the addition of the saccharide to the conditioning Shampoos B and C, respectively.

Example III

The following series of shampoos based on an amphoteric detergent were made and tested. Formulations B and C were passed through a colloid mill to assure their homogeneity.

| | % | | |
|---|---|---|---|
| Shampoo: | A | B | C |
| Miranol®2MCAS modified[1] | 10.0 | 10.0 | 10.0 |
| Hydroxypropylmethylcellulose[2] | 0.2 | 0.2 | 0.2 |
| Corn syrup (80% solids)[3] | — | — | 40.0 |
| Polydimethylsiloxane[4] | — | 2.0 | 2.0 |
| Water | to 100 | to 100 | to 100 |
| Foam volume (cc/g) | 13.4 | 12.4 | 13.7 |
| Foam viscosity (sec) | 5.0 | 5.0 | 6.1 |

(1) 2 - Cocoyl - 1 - (sodium carboxymethyl) - 1 - [2 - (sodium carboxymethoxy)ethyl] - 2 - imidazoline.
(2) As in Example I.
(3) As in Example I.
(4) As in Example I.

The results show that the loss in foam volume due to the presence of the silicone hair conditioning agent was overcome by the inclusion of the saccharide.

Example IV

The following series of shampoos were made and tested.

| | % | |
|---|---|---|
| Shampoo: | A | B |
| Sodium lauryl ether sulphate (2EO) | 11.5 | 11.5 |
| Coconut diethanolamide | 2.0 | 2.0 |
| Guar hydroxypropyl trimethyl ammonium chloride[1] | 0.3 | 0.3 |
| Mono- and di-oleyl triethoxy phosphate mixture[2] | 1.5 | 1.5 |
| Sucrose | — | 15.0 |
| Water<br>pH 6.5—7.0 (adjusted with 0.1M sodium hydroxide) | to 100 | to 100 |
| Foam volume (cc/g) | 6.0 | 6.7 |

[1] Jaguar®C-13-S.
[2] Briphos®03D.

The Example shows that the foam volume of Shampoo A (which is generally in accordance with US Patent No. 4 298 494) is improved by the inclusion of the saccharide.

Example II

The following series of formulations were made and tested:—

| | % | | | | |
|---|---|---|---|---|---|
| Shampoo: | A | B | C | D | E |
| Alpha-olefin sulphonate[1] | 10.0 | 10.0 | 10.0 | 10.0 | 10.0 |
| Hydroxypropylmethylcellulose[2] | 0.2 | 0.2 | 0.2 | 0.2 | 0.2 |
| Cocodiethanolamide | — | — | 1.0 | — | 1.0 |
| Corn syrup (80% solids)[3] | — | — | — | 40.0 | 40.0 |
| Polydimethylsiloxane[4] | — | 2.0 | 2.0 | 2.0 | 2.0 |
| Water | to 100 | to 100 | to 100 | to 100 | to 100 |
| Foam volume (cc/g) | 9.9 | 9.5 | 9.0 | 10.2 | 10.6 |
| Foam viscosity (sec) | 4.1 | 4.6 | 4.2 | 5.3 | 5.7 |

(1) Sulphramin®AOS, Witco Chemical Corp..
(2) As in Example I.
(3) As in Example I.
(4) As in Example I.

The results for Shampoos D and E show the beneficial effect of the addition of the saccharide to the conditioning Shampoos B and C, respectively.

Example III

The following series of shampoos based on an amphoteric detergent were made and tested. Formulations B and C were passed through a colloid mill to assure their homogeneity.

| | % | | |
|---|---|---|---|
| Shampoo: | A | B | C |
| Miranol®2MCAS modified[1] | 10.0 | 10.0 | 10.0 |
| Hydroxypropylmethylcellulose[2] | 0.2 | 0.2 | 0.2 |
| Corn syrup (80% solids)[3] | — | — | 40.0 |
| Polydimethylsiloxane[4] | — | 2.0 | 2.0 |
| Water | to 100 | to 100 | to 100 |
| Foam volume (cc/g) | 13.4 | 12.4 | 13.7 |
| Foam viscosity (sec) | 5.0 | 5.0 | 6.1 |

(1) 2 - Cocoyl - 1 - (sodium carboxymethyl) - 1 - [2 - (sodium carboxymethoxy)ethyl] - 2 - imidazoline.
(2) As in Example I.
(3) As in Example I.
(4) As in Example I.

The results show that the loss in foam volume due to the presence of the silicone hair conditioning agent was overcome by the inclusion of the saccharide.

Example IV

The following series of shampoos were made and tested.

| | % | |
|---|---|---|
| Shampoo: | A | B |
| Sodium lauryl ether sulphate (2EO) | 11.5 | 11.5 |
| Coconut diethanolamide | 2.0 | 2.0 |
| Guar hydroxypropyl trimethyl ammonium chloride[(1)] | 0.3 | 0.3 |
| Mono- and di-oleyl triethoxy phosphate mixture[(2)] | 1.5 | 1.5 |
| Sucrose | — | 15.0 |
| Water<br>pH 6.5—7.0 (adjusted with 0.1M sodium hydroxide) | to 100 | to 100 |
| Foam volume (cc/g) | 6.0 | 6.7 |

[(1)] Jaguar®C-13-S.
[(2)] Briphos®03D.

The Example shows that the foam volume of Shampoo A (which is generally in accordance with US Patent No. 4 298 494) is improved by the inclusion of the saccharide.

Examples V to VII

The following are further examples of shampoos of the invention:—

| | % | | |
|---|---|---|---|
| Shampoo: | V | VI | VII |
| Triethanolamine lauryl sulphate | 16.8 | 18.0 | 16.8 |
| Coconut diethanolamide | 3.0 | — | 1.0 |
| Hydroxypropylmethylcellulose[1] | 0.25 | 0.1 | 0.3 |
| Corn syrup (80% solids)[2] | 20.5 | 40.0 | 21.0 |
| Dimethylpolysiloxane[3] | 1.0 | 1.0 | — |
| Volatile silicone[4] | — | — | 1.0 |
| Cationic cellulose[5] | 0.5 | — | 0.5 |
| Cationic guar gum derivative[6] | — | 0.3 | — |
| Ethyl alcohol (SDA 40) | 9.0 | 10.0 | 10.0 |
| Vinyl carboxy polymer[7] | 0.75 | 0.3 | 0.75 |
| Perfume, colour, preservative | q.s. | q.s. | q.s. |
| Water | | to 100 | |
| Acid or base to pH: | 6.5 | 6.5 | 6.5 |

[1] As in Example I.
[2] As in Example I.
[3] As in Example I.
[4] Dow Corning 344.
[5] Polymer JR 400.
[6] Jaguar®C-17.
[7] Carbopol®941 (BF Goodrich).

Examples VIII to XI

The following are further examples of shampoos of the invention:—

| | | % | | | |
|---|---|---|---|---|---|
| Shampoo: | | VIII | IX | X | XI |
| Ammonium lauryl sulphate | | 16.8 | — | — | — |
| Sodium lauryl sulphate | | — | 14.0 | 15.0 | — |
| Triethanolamine lauryl sulphate | | — | — | — | 17.0 |
| Lauric isopropanolamide | | 1.0 | — | — | 1.0 |
| Corn syrup (80% solids)[1] | | 15.0 | — | — | — |
| Sorbitol (70%) | | — | 30.0 | — | — |
| Hydrogenated corn syrup[2] | | — | — | 30.0 | — |
| Sucrose | | — | — | — | 20.0 |
| Methyl hydrogenated rosinate | | 5.0 | — | — | — |
| Merquat®550 | | — | 2.0 | — | — |
| Cationic cellulose[3] | | — | — | 0.7 | 0.5 |
| Polydimethylsiloxane | | — | 1.0[4] | 2.0[5] | — |
| Light mineral oil | | — | — | — | 3.0 |
| Hydroxypropylmethylcellulose[6] | | 0.75 | — | 0.75 | 0.5 |
| Methyl cellulose | | — | 0.5 | — | — |
| Formaldehyde | | — | — | 0.1 | — |
| Sodium formaldehyde bisulphite | | 0.25 | — | | 0.25 |
| Dowicil®200[7] | | — | 0.1 | | — |
| Cocomonoethanolamide | | — | — | 1.5 | — |
| Cocodiethanolamide | | 1.0 | — | — | — |
| Ethtylenediamine tetraacetate | | — | — | 0.1 | — |
| Ethyl alcohol (SDA 40) | | 10.0 | 10.0 | 8.0 | 10.0 |
| Perfume, colour | | q.s. | q.s. | q.s. | q.s. |
| Water | | | to 100 | | |
| Acid or base | to pH: | 7 | 7 | 7 | 7 |

[1] As in Example I.
[2] Lycasin® (Roquette Frères).
[3] Polymer JR 400 (Union Carbide Corp.).
[4] As in Example I.
[5] 1,000 Centistokes (Viscasil®, General Electric Co.).
[6] As in Example I.
[7] CTFA Designation Quaternium 15 (Dow Chemical Co.).

**Claims**

1. An aqueous liquid hair conditioning shampoo composition comprising:
(a) from 3 to 60% by weight of an anionic or amphoteric detergent or a mixture thereof;
(b) from 0.1 to 30% by weight of at least one cationic or non-ionic hair conditioning agent;
(c) from 20 to 75% by weight water; and
(d) from 10 to 70% by weight of a saccharide chosen from mono-, di- and polysaccharides, their hydrogenation products and mixtures thereof, which are completely miscible with water in that a mixture of 70 parts by weight saccharide and 30 parts by weight water will yield an essentially clear solution which is pourable at 0°C or higher.

2. A shampoo composition as claimed in Claim 1 wherein the saccharide is a hydrolysed corn starch or hydrolysed potato starch.

3. A shampoo composition as claimed in Claim 1 or Claim 2, wherein the concentration of saccharide in the shampoo is from 15 to 60% by weight.

4. A shampoo composition as claimed in any of the preceding claims wherein the hair conditioning agent is a cationic cellulose ether or a cationic guar gum derivative.

5. A shampoo composition as claimed in any of the preceding claims wherein the hair conditioning agent consists of or includes a linear or cyclic polydimethyl siloxane.

**Patentansprüche**

1. Eine wäßrig flüssige Haarkonditionierungsshampoo-Zusammensetzung enthaltend:
(a) 3 bis 60 Gew.% eines anionischen oder amphoteren Reinigungsmittels oder einer Mischung davon;
(b) 0.1 bis 30 Gew.% mindestens eines kationischen oder nichtionischen Haarkonditionierungsmittels;
(c) 20 bis 75 Gew.% Wasser; und
(d) 10 bis 70 Gew.% eines Saccharids, ausgewählt aus Mono-, Di- und Polysacchariden, ihren Hydrierungsprodukten und Mischungen davon, die mit Wasser vollständig mischbar sind, indem eine Mischung von 70 Gew. Teilen Saccharid und 30 Gew. Teilen Wasser eine im wesentlichen klare Lösung liefert, die bei 0°C oder höher geißbar ist.

2. Eine Shampoozusammensetzung wie in Anspruch 1 beansprucht, worin das Saccharid eine hydrolysierte Maisstärke oder hydrolysierte Kartoffelstärke ist.

3. Eine Shampoozusammensetzung wie in Anspruch 1 oder Anspruch 2 beansprucht, worin die Saccharidkonzentration im Shampoo 15 bis 60 Gew.% beträgt.

4. Eine Shampoozusammensetzung wie in irgendeinem der vorhergehenden Ansprüche beansprucht, worin das Haarkonditionierungsmittel ein kationischer Celluloseether oder ein kationisches Guargummi-Derivat ist.

5. Eine Shampoozusammensetzung wie in irgendeinem der vorhergehenden Ansprüche beansprucht, worin das Haarkonditionierungsmittel aus einem linearen oder cyclischen Polydimethylsiloxane besteht oder es enthält.

**Revendications**

1. Composition de shampooing capillaire liquide aqueux traitant comprenant:
(a) de 3 à 60% en poids d'un détergent anionique ou amphotère ou un de leurs mélanges;
(b) de 0,1 à 30% en poids d'au moins un agent traitant capillaire cationique ou non ionique;
(c) de 20 à 75% en poids d'eau; et
(d) de 10 à 70% en poids d'un saccharide choisi parmi les mono-, di- et poly-saccharides, leurs produits d'hydrogénation et leurs mélanges, qui sont complètement miscibles à l'eau de telle manière qu'un mélange de 70 parties en poids de saccharide et de 30 parties en poids d'eau donnera une solution essentiellement claire que l'on peut verser à 0°C et au-dessus.

2. Composition de shampooing selon la revendication 1 dans laquelle le saccharide hydrolysé est un amidon de maïs hydrolysé ou un amidon de pomme de terre hydrolysé.

3. Composition de shampooing selon la revendication 1 ou la revendication 2 dans laquelle la concentration en saccharide dans le shampooing est comprise entre 15 et 50% en poids.

4. Composition de shampooing selon l'une quelconque des revendications précédentes dans laquelle l'agent de traitement capillaire est un éther de cellulose cationique ou un dérivé cationique de gomme guar.

5. Composition de shampooing selon l'une quelconque des revendications précédentes dans laquelle l'agent de traitement capillaire est constitué de, ou comprend, un polydiméthylsiloxane linéaire ou cyclique.